# EUROPEAN PATENT APPLICATION

(11) **EP 3 213 669 A1**
(43) Date of publication of application: **06.09.2017**
(21) Application number: 17156926.2
(22) Date of filing: 20.02.2017
(51) Int. Cl.: A61B 3/10, A61B 3/16

(54) **OPHTHALMIC DEVICE**

(30) Priority: 22.02.2016 JP 2016031471
(71) Applicant: Tomey Corporation, Nagoya-shi, Aichi 451-0051 (JP)
(72) Inventor: BIAN, Guangchun, Nagoya-shi, Aichi-ken,, 4510051 (JP); KAMO, Takashi, Nagoya-shi, Aichi-ken,, 4510051 (JP)
(74) Representative: Winter, Brandl, Fürniss, Hübner, Röss, Kaiser, Polte - Partnerschaft mbB

(57) **Abstract**

An ophthalmic device is provided with an illumination optical system configured to irradiate light irradiated from a light source as parallel light to a subject's eye at an oblique angle, the light source being configured to emit coherent light; an image capturing optical system comprising a light receiving element and configured to capture a reflected image by receiving first reflected light and second reflected light by the light receiving element, the first reflected light being reflected from an anterior surface of a cornea of the subject's eye, the second reflected light being different from the first reflected light and reflected from a posterior surface of the cornea of the subject's eye; and a controller configured to calculate a thickness of the cornea by using the reflected image captured by the image capturing optical system.

## Description

### CROSS-REFERENCE TO RELATED APPLICATION

This application claims priority to Japanese Patent Application No. 2016-031471 filed on February 22, 2016, the entire contents of which are hereby incorporated by reference into the present application.

### TECHNICAL FIELD

The present disclosure relates to an ophthalmic device configured to measure a thickness of a cornea (hereafter, corneal thickness) of a subject's eye.

### DESCRIPTION OF RELATED ART

Conventionally, as an ophthalmic device configured to measure a corneal thickness, an ophthalmic device as disclosed in Japanese Patent Application Publication No. 2005-253576 is known. This device measures the corneal thickness by irradiating measurement light at an oblique angle to the subject's eye and using reflected light from an anterior surface of the cornea and reflected light from a posterior surface of the cornea.

### SUMMARY

In conventional products, LED (abbreviation for Light-Emitting Diode) is typically used for measurement light for measuring the corneal thickness. However, because LED has a large light emitting size, a light projecting optical system for such LED products resultantly has had a complicated structure that includes a magnification conversion optical system where the measurement light is condensed by a light projection lens after passing through a slit and then irradiated to the cornea of the subject's eye, resulting in an increased number of components.

In light of the above mentioned circumstances, the present disclosure provides an ophthalmic device that has a simpler structure of an optical system by using a light source having a small light emitting size and configured to emit coherent light such that a slit can be done away with.

An ophthalmic device disclosed herein comprises: an illumination optical system configured to irradiate light irradiated from a light source as parallel light to a subject's eye at an oblique angle, the light source being configured to emit coherent light; an image capturing optical system comprising a light receiving element and configured to capture a reflected image by receiving first reflected light and second reflected light by the light receiving element, the first reflected light being reflected from an anterior surface of a cornea of the subject's eye, the second reflected light being different from the first reflected light and reflected from a posterior surface of the cornea of the subject's eye; and a controller configured to calculate a thickness of the cornea by using the reflected image captured by the image capturing optical system.

The light irradiated from the light source may have high spatial coherence.

The light source may be a SLD (abbreviation of Super Luminescent Diode).

The image capturing optical system may comprise a speckle noise reducer configured to reduce speckle noise of the reflected image.

The light receiving element may be a one-dimensional light receiving element.

The light receiving element may be a two-dimensional light receiving element.

As mentioned above, the ophthalmic device disclosed herein has a simple structure, and is configured to measure a corneal thickness using optical systems allowing the number of components to be reduced and a system thereof to be downsized.

### BRIEF DESCRIPTION OF DRAWINGS

FIG. 1 illustrates a schematic configurational diagram of an optical system of an ophthalmic device according to one embodiment of the present disclosure;
FIG. 2 illustrates a block diagram of a control system of the ophthalmic device;
FIG. 3 is a flowchart of an operation flow of the ophthalmic device;
FIG. 4 illustrates data on light received using a one-dimensional light receiving element in a corneal thickness examination optical system of the ophthalmic device; and
FIG. 5 illustrates an image captured using a two-dimensional light receiving element in the corneal thickness examination optical system of the ophthalmic device.

### DETAILED DESCRIPTION

### EMBODIMENT

Hereinbelow, an ophthalmic device according to one embodiment of the present disclosure will be explained with reference to the accompanying drawings.

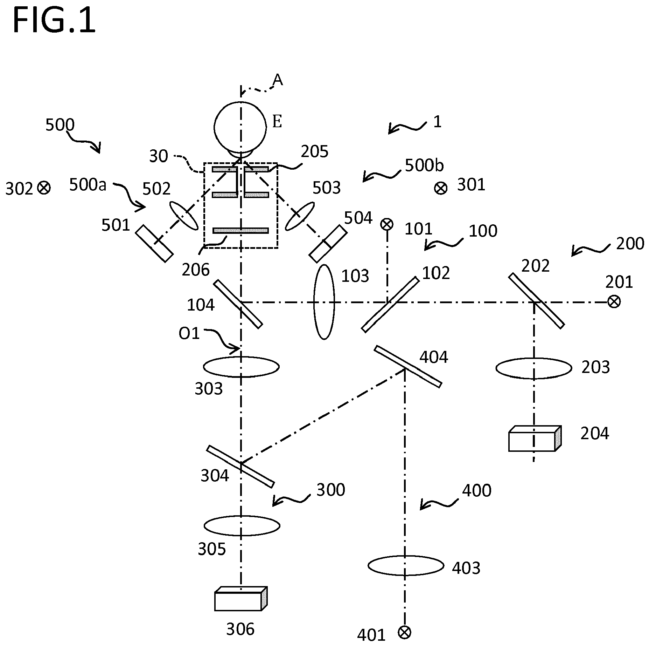

FIG. 1 is a diagram describing details of an optical system of an ophthalmic device 1 according to the present disclosure. Further in the present embodiment, the ophthalmic device 1 is configured to measure a corneal thickness and intraocular pressure (i.e., tonometry) in a non-contact manner.

The optical system of the ophthalmic device 1 comprises: an XY alignment optical system 100, an observation optical system 300, a fixation optical system 400, a tonometry optical system 200, and a corneal thickness examination optical system 500. The XY alignment optical system 100 is constituted of components from a light source 101 to a hot mirror 104. The observation optical system 300 is constituted of components from light sources 301 and 302 to a two-dimensional image sensor (CCD) 306. The fixation optical system 400 is constituted of components from a light source 401 to a mirror 404. The tonometry optical system 200 is constituted of components from a light source 201, a nozzle 205, to a flat glass 206, and configured to detect a degree of corneal deformation of a subject's eye E. The corneal thickness examination optical system 500 comprises an illumination optical system 500a constituted of components from a light source 501 to a lens 502, and an imaging optical system 500b constituted of components from a columnar lens 503 to a light receiving element 504. Further, the ophthalmic device 1 comprises an examination window 30 which is rotatable and the nozzle 205 and the flat glass 206 are housed in.

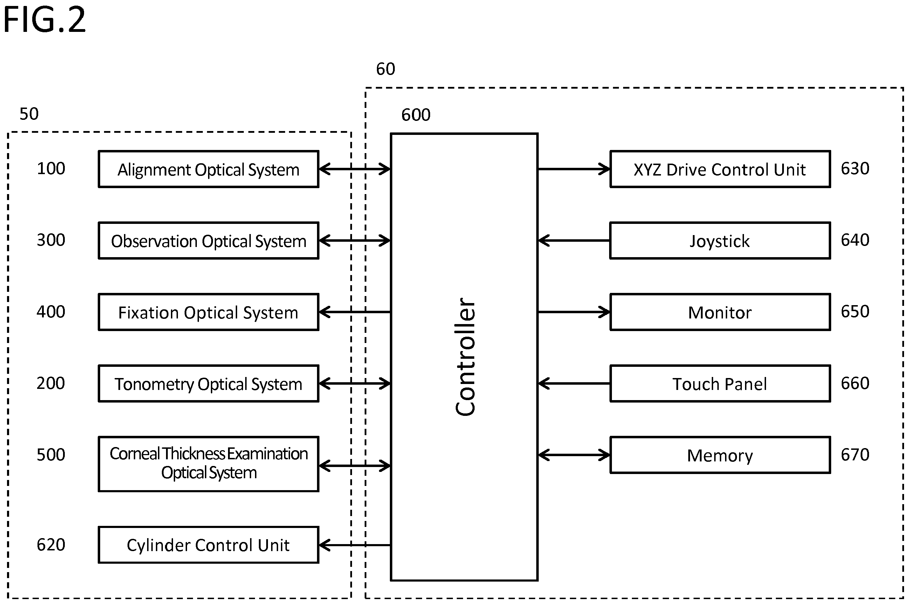

As shown in FIG. 2, the ophthalmic device 1 comprises a head section 50 in which the optical systems configured to examine the subject's eye E are disposed, and a main body 60 which is configured to control the optical systems and the like disposed in the head section 50 and comprises a monitor 650 etc. that displays a captured image of an anterior segment and inspection results. At a time of an examination, the head section 50 is moved in X, Y, and Z directions (side to side, up and down, and back and forth directions) relative to the main body 60 to examine the subject's eye E.

### (XY Alignment Optical System 100)

In the XY alignment optical system 100, light from the light source 101 is reflected by a hot mirror 102, passes through an objective lens 103, and is reflected by the hot mirror 104. Then, the light passes through the flat glass 206 and an opening of the nozzle 205, and is irradiated to a cornea of the subject's eye E. In the present embodiment, an LED which emits infrared light is implemented as the light source 101.

Light reflected by the cornea is received by the two-dimensional image sensor (CCD) 306 disposed on a main optical axis O1. The controller 600 of the main body 60 processes signals obtained in the two-dimensional image sensor (CCD) 306, and causes an XYZ drive control unit 630 to conduct an XY alignment (fine adjustment) of the head section 50 relative to the subject's eye E. Although described later in detail, the alignment of the head section 50 relative to the subject's eye E is controlled such that an examiner sees a bright spot created by alignment light on an anterior segment image which is displayed on the monitor 650 and conducts a rough alignment by operating a joystick 640 provided in the main body 60, and when the bright spot enters a predetermined range, upon which the XYZ drive control unit 630 is caused to conduct an XY automatic alignment.

### (Observation Optical System 300)

In the observation optical system 300, an anterior segment region of the subject's eye E including the cornea of the subject's eye E is irradiated by the light sources 301 and 302 which are disposed on a subject's eye E side of the head section 50. An image of the anterior segment of the subject's eye E is obtained by an objective lens 303, an imaging lens 305, and the two-dimensional image sensor (CCD) 306, and the obtained anterior segment image of the subject's eye E is displayed on the monitor 650. Although LEDs which emit infrared light are implemented as the light sources 301 and 302, they may employ other varieties of light of which wavelength is shorter than that of the alignment light source 101. Accordingly, the hot mirror 104 allows the light for observation (observation light) to penetrate therethrough, and reflects thereon the light for alignment (alignment light, light from the light source 101). Further, a dichroic mirror 304 is configured to have its reflecting and penetrating wavelength ranges set so as to allow the observation light to penetrate therethrough. Consequently, the alignment light and the observation light are appropriately separated, enabling measurements thereof to be conducted respectively.

### (Fixation Optical System 400)

In the fixation optical system 400, light from the light source 401 (fixation light) passes through a relay lens 403, and is reflected by the reflecting mirror 404. Then, the light is reflected by the dichroic mirror 304, travels along the main optical axis O1, passes through the objective lens 303 and the hot mirror 104, and forms an image on the cornea of the subject's eye E. Therefore, it is preferable that positions of the light source 401 and the cornea of the subject's eye E are approximately conjugate to each other. The subject's eye E is caused to fixate according to the fixation light, enabling an examination of an eye characteristic such as tonometry. An LED which emits visible light which the subject can see is implemented as the light source 401.

### (Tonometry Optical System 200)

In the tonometry optical system 200, a part of light from the light source 201 (deformation detecting light) penetrates a half mirror 202. Then, the light penetrates the hot mirror 102 and the objective lens 103, is reflected by the hot mirror 104, travels along the main optical axis O1, passes through the flat glass 206 and the opening of the nozzle 205, and is irradiated to the cornea of the subject's eye E. The light irradiated to the cornea is reflected by the cornea, and on its reversed route, it passes through the opening of the nozzle 205 and the flat glass 206, is reflected by the hot mirror 104, and passes through the objective lens 103 and the hot mirror 102. Then a part of the light is reflected by the half mirror 202, and received by a condenser lens 203 at a light receiving element 204. Although described later in detail, at the time of tonometry, compressed air is puffed toward the cornea of the subject's eye E from the nozzle 205 by a cylinder control unit 620 being driven by the controller 600. Since the cornea is displaced and deformed when the air is puffed thereon, an amount of light received by the light receiving element 204 changes. An intraocular pressure value of the subject's eye E is calculated according to a degree of the change in the light amount. Although an LED which emits infrared light is also implemented as the light source 201, it may employ other varieties of light of which wavelength is longer than that of the observation light and shorter than that of the alignment light. As such, by setting the wavelength of each of the alignment light, the observation light, the fixation light, and the deformation detecting light (light from the light source 201), and setting reflection/penetration characteristics of the hot mirrors 102, 104, and the dichroic mirror 304 appropriately, each of those four lights is configured to travel along an appropriate corresponding optical path.

### (Corneal Thickness Examination Optical System 500)

In the corneal thickness examination optical system 500, light from the light source 501 that has become parallel light after passing through the lens 502 is irradiated onto the cornea of the subject's eye E. It should be noted that the illumination optical system 500a including the light sources 501 and 502 has a light axis that extends obliquely relative to the subject's eye E (in detail, eye axis A). Light reflected at an anterior surface of the cornea and light reflected at a posterior surface of the cornea respectively pass through the columnar lens 503 to become linear light, which is received by the light receiving element 504. A super luminescent diode (SLD) configured to emit light having coherence and high spatial coherence is implemented as the light source 501. The light emitted from the light source 501 is infrared light of which center wavelength is 700-1000nm. The spatial coherence is an index for indicating degree of phase alignment in a plane perpendicular to a light axis. Generally, the light emitted from the SLD has a higher spatial coherence than a spatial coherence of the light emitted from an LED. Due to this, there is no need for a slit to be arranged between the light source 501 and the lens 502 in the illumination optical system 500a. Due to this, the optical system can be structured simpler compared to the case where LED is used as the light source. Moreover, it should be noted that not being limited to the SLD, other light sources such as laser diode (LD) may be implemented as the light source 501 as long as the light source 501 is configured to emit coherent light with high spatial coherence.

If a light source configured to emit light having coherence is used as the light source here, speckle noise occurs, degrading measurement accuracy of the corneal thickness. However, the speckle noise can be reduced by allowing the lights reflected at the corneal anterior surface and the posterior surface of the subject's eye E to pass through the columnar lens 503 to be the linear light.

Further, the corneal thickness examination optical system 500 also functions as a Z alignment optical system. The controller 600 of the main body 60 processes the reflected light from the cornea obtained at the light receiving element 504, and causes the XYZ drive control unit 630 to conduct a Z alignment (fine adjustment) of the head section 50 relative to the subject's eye E. Although described later in detail, the alignment of the head section 50 relative to the subject's eye E is controlled such that the examiner sees the bright spot of alignment light on the anterior segment image which is displayed on the monitor 650 and conducts the rough alignment by operating the joystick 640 provided in the main body 60, and when the bright spot enters the predetermined range, upon which the XYZ drive control unit 630 is caused to conduct the Z automatic alignment.

The reason why the light from the light source 501 is made to pass through the lens 502 so as to be the parallel light is an attempt to suppress fluctuation in data obtained by the Z alignment. If light condensed to an apex of the cornea is used instead of the parallel light, the image obtained at the light receiving element 504 would be blurred when the light is not condensed to the corneal apex but displaced in the Z direction (back and forth direction), resulting in degraded accuracy of measured results.

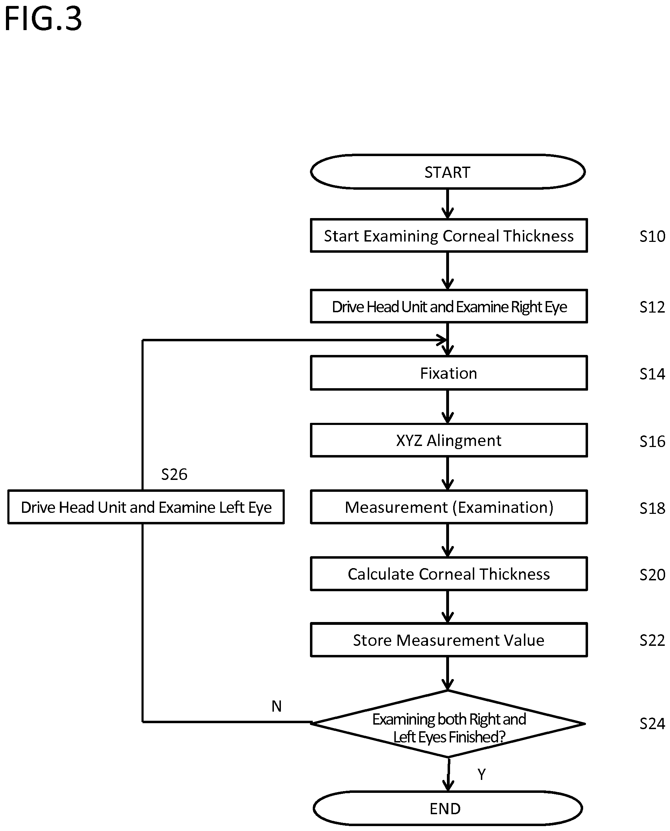

Next, how the corneal thickness is measured in the ophthalmic device 1 configured as above will be described. FIG. 3 illustrates a flow chart of the corneal thickness measurement procedures.

In S10, in response to the examiner operating a touch panel 660, the controller 600 starts the examination of the corneal thickness. Although not described in the operation flow, at this occasion, the observation light sources 301, 302, the alignment light source 101, the fixation light source 401, and the corneal thickness examination light source 501 are turned on.

In S12, the examiner operates the joystick 640 to move the head section 50 such that a right eye of a patient is displayed on the monitor 650. Then according to an operation by the examiner, the head section 50 is roughly aligned in the X, Y, and Z directions such that the bright spot on the cornea enters the predetermined range.

In S14, a fixation lighting from the light source 401 fixates the subject's eye E. It should be noted that the light source 401 may be moved by using a result of an eye refraction test (examination for eye refractive power) to be substantially conjugate with the retina of the subject's eye E. Due to this, the fixation lighting can be arranged at a position in focus relative to the patient. The result of the eye refraction test may be a result measured by an ophthalmic device including an eye refractive power optical system in addition to the present embodiment optical systems, or may be a result measured by an ophthalmic device including different optical systems than those of the present embodiment.

In S16, the controller 600 detects the state of the alignment based on signals obtained by the two-dimensional image sensor (CCD) 306 and the light receiving element 504, and based on detected results, the XYZ alignment of the head section 50 is then conducted by the XYZ drive control unit 630 of the main body 60.

Once the XYZ alignment has been complete, the controller 600 starts measurement in S18. As mentioned above, the controller 600 irradiates the light from the light source 501 onto the cornea of the subject's eye E and receives reflected light therefrom at the light receiving element 504.

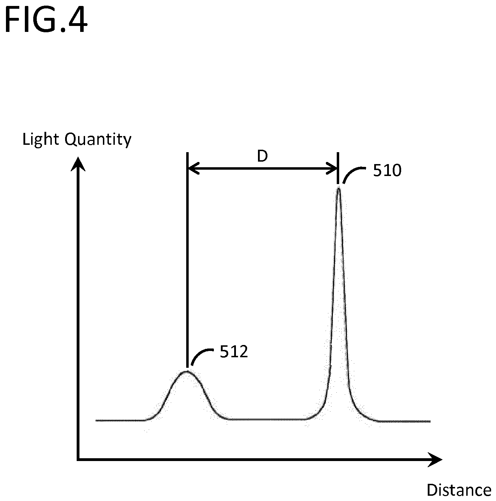

Subsequently, the controller 600 calculates the corneal thickness in S20. FIG. 4 illustrates waveform data received by using a one-dimensional light receiving element in the corneal thickness examination optical system 500. In FIG. 4, a first peak 510 having a greatest light amount indicates the reflected light from the corneal anterior surface. A second peak 512 having a second greatest light amount indicates the reflected light from the corneal posterior surface. A corneal thickness D is calculated from a difference in positions between the two peaks. Notably, as the one-dimensional light receiving element, a line sensor for example may be used. When the one-dimensional light receiving element is used as the light receiving element 504, the corneal thickness examination optical system 500 can be configured inexpensively as compared to a case a two-dimensional light receiving element (described later in detail) is used.

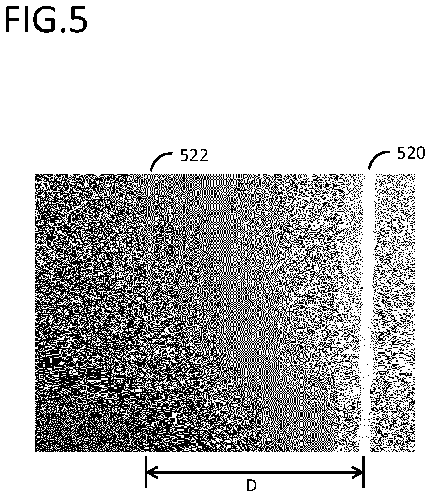

FIG. 5 indicates an image captured using the two-dimensional light receiving element in the corneal thickness examination optical system 500. The controller 600 calculates the corneal thickness D by analyzing luminance information of the captured image and using a difference in positions between a first peak 520 and a second peak 522 that have local maximum values of luminance. Notably, as the two-dimensional light receiving element, a CCD camera or a profile sensor for example may be used.

In S22, the controller 600 stores the measured values in a memory 670.

In S24, the controller 600 determines whether both left and right eyes have been examined. When the right eye has only been examined, in S26 the controller 600 moves the head section 50 to a left eye side, and similarly to the right eye, the corneal thickness D of the left eye is measured in S 14 to S22 and the measured value is stored in the memory 670.

Upon completion of the measurement of both the right and left eyes, the measurement ends.

It should be noted that in the present embodiment, the examination is started with the right eye, however, the examination may be started with the left eye, or only one eye may be examined.

While specific examples of the present disclosure have been described above in detail, these examples are merely illustrative and specific descriptions place no limitation on the scope of the present disclosure. It should be understood that the present disclosure may include various changes which do not depart from the spirit and scope of the present disclosure.

For example, in S20 of FIG. 3, although the positions at which the light amount takes the peak value in the data obtained by the one-dimensional light receiving element or the positions at which the luminance of the image data obtained by the two-dimensional light receiving element takes the local maximum value are used as the indications for the reflected lights from the corneal anterior surface and the corneal posterior surface to calculate the corneal thickness D, alternatively, half width midpoints may be determined as reflected lights respectively from the corneal anterior surface and the corneal posterior surface. Due to this, measurement errors can be suppressed when the data around the peak values are fluctuated due to noise or corneal abnormalities.

Further, although the present embodiment discloses the configuration that conducts tonometry, the present disclosure is not limited to this. For example, the ophthalmic device disclosed herein may be configured to conduct a corneal shape examination, and/or may be configured to conduct a corneal endothelium examination. Further, the ophthalmic device disclosed herein may not be configured to conduct the tonometry but only be configured to conduct the corneal thickness examination.

## Claims

1. An ophthalmic device (1) comprising:
an illumination optical system (500a) configured to irradiate light irradiated from a light source (501) as parallel light to a subject's eye at an oblique angle, the light source being configured to emit coherent light;
an image capturing optical system (500b) comprising a light receiving element (504) and configured to capture a reflected image by receiving first reflected light and second reflected light by the light receiving element (504), the first reflected light being reflected from an anterior surface of a cornea of the subject's eye, the second reflected light being different from the first reflected light and reflected from a posterior surface of the cornea of the subject's eye; and
a controller (600) configured to calculate a thickness of the cornea by using the reflected image captured by the image capturing optical system (500b).

2. The ophthalmic device (1) as in claim 1, wherein
the light irradiated from the light source (501) has high spatial coherence.

3. The ophthalmic device (1) as in claim 1 or 2, wherein
the light source (501) is a SLD (abbreviation of Super Luminescent Diode).

4. The ophthalmic device (1) as in any one of claims 1 to 3, wherein
the image capturing optical system (500b) comprises a speckle noise reducer (503) configured to reduce speckle noise of the reflected image.

5. The ophthalmic device (1) as in any one of claims 1 to 4, wherein
the light receiving element (504) is a one-dimensional light receiving element.

6. The ophthalmic device (1) as in any one of claims 1 to 4, wherein
the light receiving element (504) is a two-dimensional light receiving element.
